# EUROPEAN PATENT APPLICATION

(11) **EP 0 921 396 A2**
(43) Date of publication of application: **09.06.1999**
(21) Application number: 98309497.0
(22) Date of filing: 19.11.1998
(51) Int. Cl.: G01N 33/48, C12Q 1/44

(54) **Methods of rapid screening of cytochrome cyp2d6 status**

(30) Priority: 08.12.1997 US 67954 P
(71) Applicant: Pfizer Products Inc., Groton, Connecticut 06340 (US)
(72) Inventor: Friedman, Hylar Lewis, Brattleboro, Vermont 05301 (US); Shah, AjitKumar Kantilal, Waterford, Connecticut 06385 (US)
(74) Representative: Hayles, James Richard

(57) **Abstract**

Mammalian subjects may be phenotyped by measuring the ratio of dextromethorphan to its metabolite dextrorphan in urine, saliva or plasma samples.

## Description

### FIELD OF THE INVENTION

This invention is directed toward methods of rapidly screening subjects for poor and extensive CYP2D6 activity.

### BACKGROUND OF THE INVENTION

There are wide inter-individual variations in drug efficacy and toxicity. To a great extent, these differences are the result of differences in the metabolism, distribution and elimination of the therapeutic agents. While clinical factors affecting distribution (body mass, albumin levels, etc.) and elimination (kidney function) are relatively easy to measure, the assessment of metabolic capacity is not a routine procedure. Part of this is due to the fact that metabolizing enzymes exhibit a large degree of inter-individual variability in their levels of expression. Some polymorphic metabolic abnormalities are understood at the DNA level (genotype) but for the majority, the underlying mutations have not been identified. In such cases, metabolic phenotyping remains the only way to assess metabolic irregularities through the identification of specific metabolite patterns produced by test compounds of `probe drugs'.

In probe drug phenotyping, urine or blood samples are collected before and after administration of the probe and if the enzyme(s) responsible for the production of a particular metabolite is (are) known, metabolite to parent ratios can be used to derive phenotypes. Individuals who are deficient in their ability to breakdown the probe drug are called `slow` or `poor` metabolizers. Those who have a normal or greater than average metabolism are called `fast` or `extensive` metabolizers.

While the genotype prescribes the native levels of enzymatic activity, changes in the relative levels and activities of metabolizing enzymes can be produced by drug interactions and/or clinical conditions such as disease progression or malnutrition. Thus a relatively healthy patient will not necessarily have the same reaction to a drug when that person's health has degenerated. This phenomenon has been suggested in AIDS patients for the activity of the enzyme N-acetyltransferase 2 (NAT2). In this case, the NAT2 phenotype appears to change in AIDS patients from "fast" to "slow" while the genotype remains constant.

The key barrier in the routine incorporation of metabolic activity assessments in clinical treatment is the lack of rapid, inexpensive and `user friendly` methods for these measurements. (Ducharme, J. of Chromat B, 678 (1996) 113-28)

The genetics of CYP2D6 have been extensively studied and this enzyme has been shown to be polymorphic. Humans can be divided into "extensive" and "poor" metabolizers with a population-based distribution which can range from only 1% poor metabolizers in Chinese subjects to a poor metabolizer frequency of 7% in a population of white Swedish subjects. Many of the large population studies and several clinical protocols have used the urinary excretion of dextrorphan (DEX) relative to dextromethorphan (DM) as the determinate of "poor" and "extensive" CYP2D6 activity. This is possible because DM and its metabolites are significantly eliminated by the kidney. Typically, the urinary molar concentration ratio of the probe drug (DM) to metabolite (DEX) over an interval of 8-10 hours is required to assign phenotype.

### SUMMARY OF THE INVENTION

Oxidative metabolism involving the cytochrome P450 2D6 (CYP2D6) is genetically determined. This results in some individuals being phenotyped as "poor" and others as "extensive" metabolizers. Typically, the urinary molar concentration ratio of a probe drug to metabolite over an interval of 8-10 hour is determined to assign phenotype. We evaluated methods to determine CYP2D6 phenotype which may be useful to rapidly screen subjects in clinical trials using dextromethorphan.

In a small group of subjects our results showed that at least a 4 hour urine collection, 2 hour plasma, or 3 hour saliva sample post-dose accurately predicted CYP2D6 phenotype.

This invention provides method for phenotyping a subject as a "poor" or "extensive" metabolizer which comprises:
a) administering a dose of dextromethorphan to said subject;
b) waiting for a period of time;
c) obtaining a sample of urine, plasma or saliva from said subject;
d) measuring the concentrations of dextromethorphan and dextrorphan in said sample;

In another aspect this invention provides a method wherein said sample is a urine sample.

In another aspect this invention provides a method wherein said sample is a saliva sample.

In another aspect this invention provides a method wherein said sample is a plasma sample.

In another aspect this invention provides a method wherein said period of time is one to four hours.

In another aspect this invention provides a method wherein said dose of dextromethorphan is 60/mg of dextromethorphan HCl.

### DETAILED DESCRIPTION OF THE INVENTION

The liver contains enzymes that convert various drug substances to products, called metabolites, which can be more easily eliminated from the body, usually in the urine or feces. This conversion process, which is also known as chemical metabolism or chemical biotransformation, frequently determines the duration of action of drugs or the intensity of the drug action, which is why drugs must typically be taken several times each day to treat diseases and produce other desirable pharmacological effects.

The many pharmaceutical-metabolizing enzyme systems of the liver include cytochrome P450, carboxylesterases, UDP-glucuronosyltransferases, sulfotransferases, glutathione S-transferases and many others. Each of these enzyme systems is comprised of numerous individual enzymes, each of which is capable of metabolizing a wide variety of pharmaceuticals and other chemical compositions. For example, the cytochrome P450 system in the human liver is comprised of at least ten individual P450 enzymes. Of these various enzyme systems, the P450 enzymes play the most important role in determining the rate of elimination of drugs.

Metabolism by cytochrome P450 often represents the rate-limiting step in pharmaceutical elimination. Consequently, factors that lessen the activity of P450 enzymes usually prolong the effects of pharmaceuticals, whereas factors that increase cytochrome P450 activity have the opposite effect.

Changes in drug metabolism may have undesirable or toxic consequences. For example, impaired metabolism of a drugs by factors that decrease cytochrome P450 activity have the opposite effect.

Conversely, the accelerated metabolism of a drug due to increased concentrations of cytochrome P450 can also lead to a lessening of therapeutic effect.

Information from reaction phenotyping can also be used to explain or predict adverse drug reactions that result from variances in the activity of various P450 enzymes. During reaction phenotyping, the quantity and/or kind of metabolites produced when a drug reacts with an enzyme are used to identify the existence of that enzyme in an individual. The pharmacologic or toxic effects of certain drugs can be exaggerated or compounded in a significant percentage of the population-at-large due to a genetic deficiency in a P450 enzyme.

Poor metabolizers often experience exaggerated responses to drugs at dosages that are well tolerated by normal subjects who have a better capability to metabolize the same drugs. If a drug is metabolized by cytochrome CYP2D6, the drug likely will have an exaggerated or toxic effect in individuals lacking CYP2D6. As noted, about 5% to 10% of all Caucasians have abnormally low concentrations of CYP2D6.

It is highly desirable to provide a method for identifying poor and extensive metabolizers of drugs. The present invention provides a simple and rapid method for identifying individuals who are "poor" and "extensive" metabolizers based on CYP2D6 activity.

In this method, a fasting subject is dosed with an effective amount of dextromethorphan (DM) and the ratio of DM to its metabolite dextrophan (DEX) in saliva, plasma or urine is determined by high performance liquid chromatography using a reversed phase column and a fluorescence detector. A DM\DEX ratio of less than 0.3 defines an "extensive" metabolizer while a ratio of greater than or equal to 0.3 defines a "poor" metabolizer.

### EXAMPLE 1

A total of 32 healthy male and female subjects (age 29±9 year and weight 74±13 kg, including 25 extensive and 7 poor metabolizers based on genotyping) were enrolled. After an ovemight fast subjects received a single 60mg dose of dextromethorphan hydrobromide. Serial plasma and saliva samples were obtained at: 0 (just prior to dosing), and 1, 2, 3, 4, 5, 6, 7, and 8 hour, and urine samples were collected at predose and 0-2, 2-4, 4-6, and 6-8 hour interval following the dose. Samples were assayed for dextromethorphan and metabolite, dextrorphan, by high-performance liquid chromatographic system equipped with a reversed phase column and a fluorescence detector. Dextromethorphan and total dextrorphan were extracted from human plasma, urine and saliva using a liquid-liquid extraction method with back-extraction.

An aliquot of human plasma, urine or saliva containing dextromethorphan and total dextrorphan was incubated with β-glucuronidase, then the intemal standard is added and the mixture is extracted with an organic solvent. After shaking and centrifuging, the analytes are back-extracted into an aqueous acidic solution. The resultant aqueous layer, containing the drugs and the internal standard, was then injected into a high performance liquid chromotographic system equipped with a reversed phase column and a fluorescence detector. Quantitation was performed using the peak height ratio method.

Molar metabolic ratios of dextromethorphan to dextrorphan in the urine collection inervals of 0-4 and 0-6 hour and plasma samples of 2 to 8 hour post dose were consistent with the urine 0-8 hour interval for determination of phenotype. Saliva results were generally erratic and unpredictable, however, the 3 hour salivary dextromethorphan to dextrorphan ratio did discriminate between extensive and poor metabolizers.

## Claims

1. A method for phenotyping a mammalian subject as a poor or extensive metabolizer which comprises:
a) administering a dose of dextromethorphan to said subject;
b) waiting for a period of time;
c) obtaining a sample of urine, plasma or saliva from said subject;
d) measuring the concentrations of dextromethorphan and dextrorphan in said sample.

2. A method of claim 1 wherein said sample is a saliva sample.

3. A method of claim 1 wherein said is a plasma sample.

4. A method of claim 1 wherein said sample is a urine sample.

5. A method of claim 2 wherein said period of time is three hours.

6. A method of claim 1 wherein said dose of dextromethorphan is 60 mg of dextromethorphan HCI.

7. A method of claim 4 wherein said period of time is four or six hours.
